# EUROPÄISCHE PATENTSCHRIFT

(11) **EP 0 811 590 B1**
(45) Veröffentlichungstag und Bekanntmachung des Hinweises auf die Patenterteilung: **20.07.2005**
(21) Anmeldenummer: 97108677.2
(22) Anmeldetag: 30.05.1997
(51) Int. Cl.: C07B 63/04, C07C 7/20, C07C 51/50, C08F 2/38

(54) **Verfahren zum Verdichten ethylenisch ungesättigter Monomerer**
Process for compressing ethylenically unsaturated monomers
Procédé pour comprimer les monomères éthylèniquement insaturés

(30) Priorität: 05.06.1996 DE 19622441
(43) Veröffentlichungstag der Anmeldung: 10.12.1997
(73) Patentinhaber: Basell Polyolefine GmbH, 50389 Wesseling (DE)
(72) Erfinder: Sutoris, Heinz Friedrich, Dr., 67227 Frankenthal (DE); Aumüller, Alexander, Dr., 67435 Neustadt (DE); Koch, Andreas, Dr., 67240 Bobenheim-Roxheim (DE); Deckers, Andreas, Dr., 55234 Flomborn (DE); Schauss, Eckard, Dr., 67259 Heuchelheim (DE); Klimesch, Roger, Dr., 64665 Alsbach-Hähnlein (DE); Kingma, Arend Jouke, Dr., 67069 Ludwigshafen (DE); Weber, Wilhelm, Dr., 67435 Neustadt (DE)

(56) Entgegenhaltungen:
- EP-A- 0 178 168
- EP-A- 0 467 848
- EP-A- 0 467 849
- EP-A- 0 467 850
- DE-A- 19 510 184
- US-A- 4 721 761
- US-A- 5 449 724

## Beschreibung

Gegenstand der vorliegenden Erfindung ist ein Verfahren zum Verdichten von ethylenisch ungesättigten Monomeren auf einem Druck zwischen 200 und 5000 bar in Abwesenheit eines Polymerisationsinitiators.

Weiterhin sind Gegenstand der vorliegenden Erfindung ein Verfahren zur Herstellung von Copolymeren durch entsprechende Verdichtung und anschließende Polymerisation sowie die Verwendung von Derivaten sterisch gehinderter Amine in einem solchen Verdichtungsverfahren.

Derivate sterisch gehinderter Amine sind als Stabilisatoren von Kunststoffen und von radikalisch polymerisierbaren Monomeren seit langem bekannt.

Aus EP-A-178 168 ist ein Verfahren zur Inhibierung von α,β-ethylenisch ungesättigten Monocarbonsäuren, beispielsweise von Acrylsäure, bei deren Aufarbeitung durch Destillation bekannt.

In US-5 449 724 wird ein Verfahren zur Herstellung thermoplastischer Ethylenhomo- und -copolymerer bei Temperaturen von 40 bis 500°C und einem Druck zwischen 500 und 5000 bar in Gegenwart eines Radikalbildners und einer stabilen freien Radikalverbindung beschrieben. Die Anwesenheit der stabilen freien Radikalverbindung, insbesondere von Derivaten des 2,2,6,6-Tetramethylpiperidin-N-oxyls, führt dabei zu einer besonders engen Molekulargewichtsverteilung und damit verbunden zu besonderen physikalischen Eigenschaften der Polymere.

US-A-4 721 761 offenbart die Verhinderung von Polymerisation von Comonomeren in Ultrahochdruck pumpen.

Bei der Verdichtung von ethylenisch ungesättigten Monomeren auf einen hohen Druck zwischen 500 und 5000 bar, insbesondere bei der Verdichtung von Monomerengemischen aus Ethylen und Acrylsäure bzw. Acrylsäurederivaten treten schon vor der Initiierung der Polymerisation in den Verdichtern und Vorverdichtern häufig vorzeitige Polymerisationen auf, die zu Belagbildungen führen und ein regelmäßiges Reinigen der Verdichter in kurzen Abständen erforderlich machen. Der Einsatz üblicher Inhibitoren wie Methylhydrochinon und Hydrochinon, zeigt nur geringe Inhibitionswirkung und erfordert hohe Inhibitorkonzentrationen.

Aufgabe der vorliegenden Erfindung war es daher, ein Verfahren zur Verdichtung von ethylenisch ungesättigten Monomeren zu finden, welches die auf vorzeitige Polymerisation zurückzuführende Belagbildung bei der Verdichtung vermindert.

Demgemäß wurde ein Verfahren zum Verdichten von ethylenisch ungesättigten Monomeren auf einem Druck zwischen 200 und 5000 bar in Abwesenheit eines Polymerisationsinitiators gefunden, welches dadurch gekennzeichnet ist, daß die Verdichtung in Gegenwart eines sterisch gehinderten Aminderivats vorgenommen wird.

Nach dem erfindungsgemäßen Verfahren können alle üblichen ethylenisch ungesättigten Monomeren verdichtet werden. Als Monomere kommen beispielsweise Ethylen, Propylen, Buten und Butadien sowie Vinylester von C₂-C₁₈-Alkancarbonsäuren wie Vinylacetat und Vinylpropionat, C₂-C₁₈-Alkylester von Acryl- bzw. Methacrylsäure wie Methyl-, Ethyl-, Propyl-, Butyl- und 2-Ethylhexylacrylat und -methacrylat, Ester monoethylenisch ungesättigter Dicarbonsäuren wie Mono- und Diester der Malein- und Fumarsäure, monoethylenisch ungesättigte Carbonsäuren wie Acrylsäure, Methacrylsäure, Maleinsäure und Fumarsäure, Amide monoethylenisch ungesättigter Carbonsäuren wie Acrylamid, Methacrylamid, N-Mono-(C₁-C₁₈)-alkylacrylamid, N-Mono-(C₁-C₁₈)-alkylmethacrylamid, N-Di-(C₁-C₁₈)-alkylacrylamid und N-Di- (C₁-C₁₈)-alkylmethacrylamid, monoethylenisch ungesättigte Alkohole, C₁-C₄-Alkylvinylether und N-vinylheterocyclische Verbindungen wie N-Vinylpyrrolidon, N-Vinylcaprolactam und N-Vinylimidazole sowie N-Vinylformamid in Betracht.

Besonders geeignet ist das erfindungsgemäße Verfahren zur Verdichtung von Monomerengemischen, wie sie zur Copolymerisation eingesetzt werden. Als Monomerengemische kommen insbesondere solche in Betracht, die neben Ethylen, Propylen, Buten oder Butadien die oben genannten Comonomere enthalten.

Als besonders geeignete Comonomere sind Derivate der Acrylsäure oder Methacrylsäure sowie diese Säuren selbst zu nennen. Geeignete Derivate dieser Säuren sind beispielsweise die C₁-C₁₈-Alkylester, C₁-C₁₈-Mono- und Dialkylamide sowie die unsubstituierten Amide. Dabei kommen als C₁-C₁₈-Alkylgruppen z.B. Methyl, Ethyl, n-Propyl, Isopropyl, n-Butyl, Isobutyl, sec-Butyl, tert-Butyl, n-Pentyl, Isopentyl, sec-Pentyl, tert-Pentyl, Neopentyl sowie die verschiedenen isomeren Hexyl-, Heptyl-, Octyl-, Nonyl-, Decyl-, Undecyl-, Dodecyl-, Tridecyl-, Tetradecyl-, Pentadecyl-, Hexadecyl-, Heptadecyl- und Octadecylreste in Betracht.

Ganz besonders bevorzugt lassen sich nach dem erfindungsgemäßen Verfahren Gemische aus Ethylen und Acrylsäure bzw. Methacrylsäure verdichten.

Erfindungsgemäß wird die Verdichtung der ethylenisch ungesättigten Monomeren in Gegenwart eines sterisch gehinderten Aminderivats vorgenommen. Unter sterisch gehinderten Aminen sollen dabei alle sekundären Amine verstanden werden, die an den zum Aminstickstoff benachbarten C-Atomen so substituiert sind, daß sie dort kein Wasserstoffatom tragen. Bevorzugt sind Derivate des 2,2,6,6-Tetramethylpiperidins, die entweder an der 4-Position oder am Aminstickstoff substituiert sind.

Bevorzugte Derivate sterisch gehinderter Amine sind die Hydroxylamine. Besonders bevorzugte Derivate sterisch gehinderter Amine sind die N-Oxyle.

Geeignete N-Oxyle von Aminen sind z.B. die folgenden Strukturen 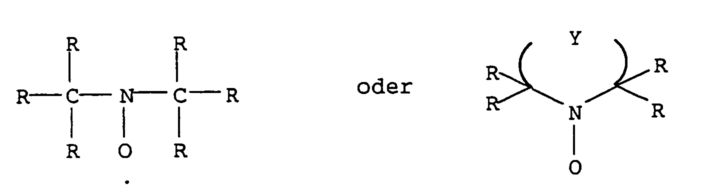 wobei R gleiche oder verschiedene Alkyl-, Cycloalkyl-, Aralkyl- oder Arylreste, die auch paarweise zu einem Ringsystem verbunden sein können, und Y eine Gruppe, die erforderlich ist, um einen 5- oder 6-gliedrigen Ring zu vervollständigen, bedeuten. Beispielsweise steht R für einen C₁-C₂₀-, insbesondere C₁-C₈-Alkylrest, einen C₅- oder C₆-Cycloalkylrest, einen Benzylrest oder einen Phenylrest. Y ist beispielsweise eine Alkylengruppe -(CH₂)₂- oder -(CH₂)₃-.

Weiterhin kommen auch N-Oxylverbindungen wie die folgenden Strukturen in Betracht 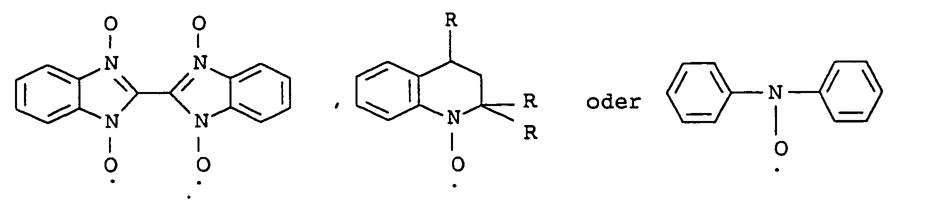 wobei die aromatischen Ringe jeweils noch 1 bis 3 inerte Substituenten tragen können, wie z.B. C₁-C₄-Alkyl, C₁-C₄-Alkoxy oder Cyano.

Vorzugsweise werden sterisch gerinderte Aminderivate von cyclischen Aminen eingesetzt, z.B. von Piperidin- oder Pyrrolidinverbindungen, die im Ring ein weiteres Heteroatom wie Stickstoff, Sauerstoff oder Schwefel enthalten können, wobei dieses Heteroatom nicht in Nachbarstellung zum gehinderten Aminstickstoff steht. Die sterische Hinderung ist durch Substituenten in beiden Nachbarstellungen zum Aminstickstoff gegeben, wobei als Substituenten Kohlenwasserstoffreste in Betracht kommen, die alle 4 Wasserstoffatome der α-CH₂-Gruppen ersetzen. Beispielsweise seien als Substituenten Phenyl, C₃-C₆-Cycloalkyl, Benzyl und insbesondere C₁-C₆-Alkylreste genannt, wobei die an dem selben α-C-Atom gebundenen Alkylreste auch untereinander zu einem 5- oder 6-Ring verbunden sein können. Besonders bevorzugt sind die unter R¹ und R² im einzelnen aufgeführten Reste. Vorzugsweise werden als N-Oxyle sterisch gehinderter Amine Derivate des 2,2,6,6-Tetraalkylpiperidins eingesetzt.

Bevorzugte N-Oxyl-Verbindungen in den erfindungsgemäßen Monomerenzusammensetzungen sind solche der allgemeinen Formel II 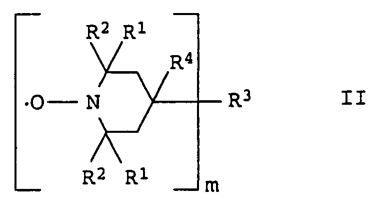 wobei
- R¹, R²: C₁-C₄-Alkyl, Phenyl oder gemeinsam mit dem C-Atom, an das sie gebunden sind, einen 5- oder 6-gliedrigen gesättigten Kohlenwasserstoffring,
- R³: Wasserstoff, Hydroxyl, Amino oder einen m-wertigen über Sauerstoff oder Stickstoff gebundenen organischen Rest oder zusammen mit R⁴ Sauerstoff oder eine unter R⁴ definierte Ringstruktur,
- R⁴: Wasserstoff, C₁-C₁₂-Alkyl oder zusammen mit R³ Sauerstoff oder zusammen mit R³ und dem C-Atom, an das sie gebunden sind, folgende Ringstrukturen 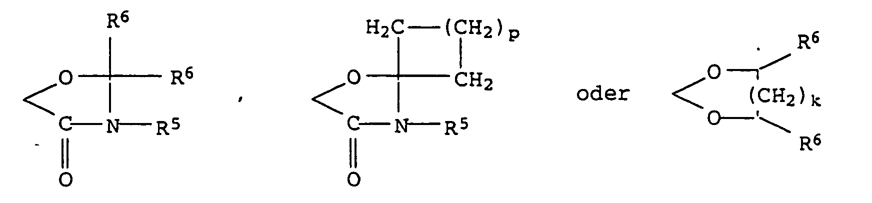 wobei für die Fälle, in denen R³ mit R⁴ einen gemeinsamen Rest bildet, m = 1 ist,
- R⁵: Wasserstoff, C₁-C₁₂-Alkyl oder -(CH₂)_{z}-COOR⁶,
- R⁶: gleiches oder verschiedenes C₁-C₁₈-Alkyl,
- k: 0 oder 1
- z, p: 1 bis 12 und
- m: 1 bis 100
bedeuten.

R¹ und R² können z.B. die C₁-C₄-Alkylgruppen Methyl, Ethyl, n-Propyl, Isopropyl, n-Butyl, Isobutyl, sec-Butyl oder tert-Butyl sein oder sie können zusammen eine Tetra- oder Pentamethylengruppe bilden. Bevorzugt sind R¹ und R² Methylgruppen.

Als R⁴ kommen beispielsweise Wasserstoff, die oben genannten C₁-C₄-Alkylgruppen sowie Pentyl, sec-Pentyl, tert-Pentyl, Neopentyl, Hexyl, 2-Methylpentyl, Heptyl, 2-Methylhexyl, Octyl, Isooctyl, 2-Ethylhexyl, Nonyl, 2-Methylnonyl, Isononyl, 2-Methyloctyl, Decyl, Isodecyl, 2-Methylnonyl, Undecyl, Isoundecyl, Dodecyl und Isododecyl, (die Bezeichnungen Isooctyl, Isononyl und Isodecyl sind Trivialbezeichnungen und stammen von den nach der Oxosynthese erhaltenen Carbonylverbindungen ab; vgl. dazu Ullmann's Encyklopedia of Industrial Chemistry, 5th Edition, Vol. A1. Seiten 290-293, sowie Vol. A10, Seiten 284 und 285) in Betracht.

p ist bevorzugt 6-12, besonders bevorzugt 9.

z ist bevorzugt 1-4, besonders bevorzugt 2.

Als R⁵ kommen neben Wasserstoff beispielsweise die oben angegebenen C₁-C₁₂-Alkylgruppen in Betracht. Bevorzugt steht R⁵ für Wasserstoff, C₁-C₄-Alkyl oder (CH₂)_{z}-COO(C₁-C₆-Alkyl), besonders bevorzugt für die Reste -CH₂-CH₂-COO(CH₂)₁₁-CH₃ und -CH₂-CH₂-COO(CH₂)₁₃-CH₃.

R⁶ kann beispielsweise eine der oben genannten C₁-C₁₂-Alkylgruppen oder Tridecyl, Isotridecyl, Tetradecyl, Pentadecyl, Hexadecyl, Heptadecyl oder Octadecyl sein. Bevorzugt sind Dodecyl und Hexadecyl.

Bevorzugte Reste R³ sind beispielsweise die folgenden m-wertigen Reste 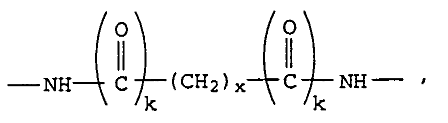 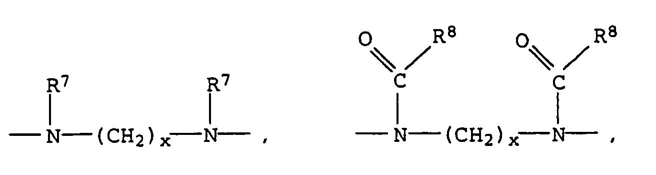 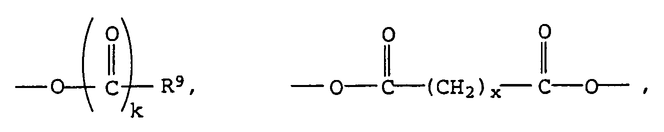 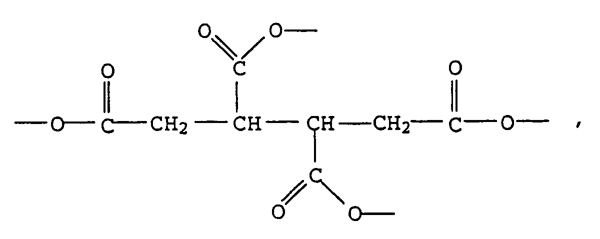 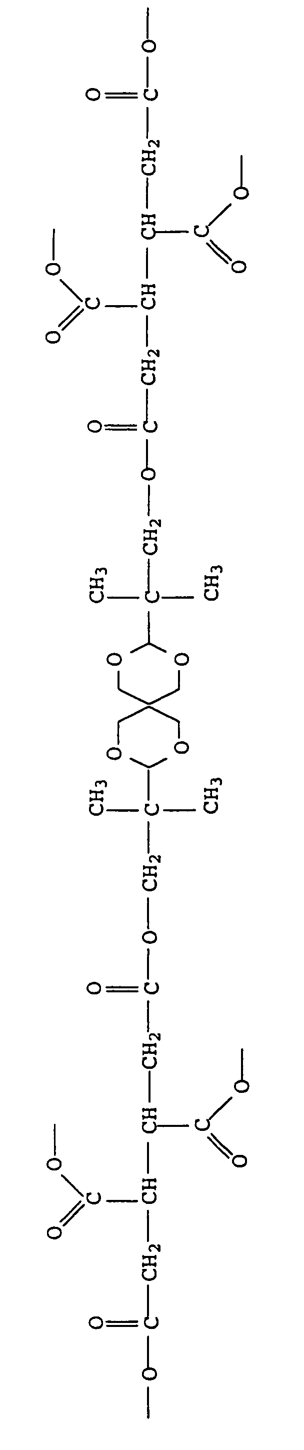 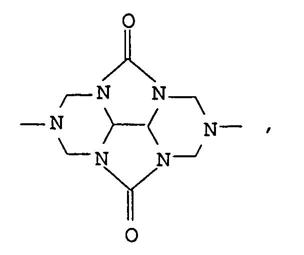 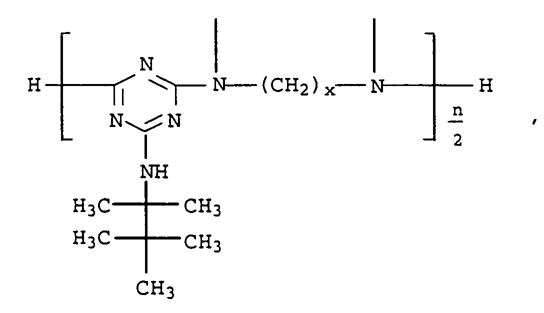 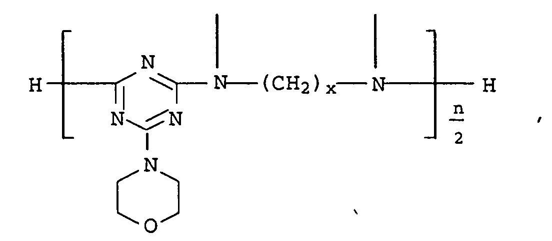 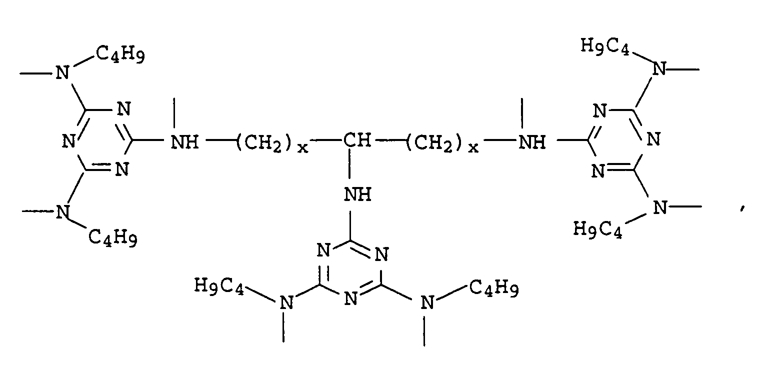 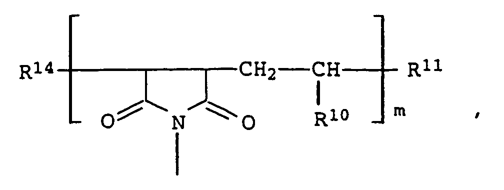 wobei
- R⁷: C₁-C₁₂-Alkyl oder -(CH₂)_{z}-COOR⁶
- R⁸: Wasserstoff oder C₁-C₁₈-Alkyl,
- R⁹: C₁-C₁₈-Alkyl, Vinyl oder Isopropenyl,
- R¹⁰: C₈-C₂₂-Alkyl,
- R¹¹: Wasserstoff oder einen organischen Rest, wie er bei der radikalischen Polymerisation der Ausgangsmonomeren üblicherweise entsteht,
- k: 0 oder 1,
- x: 1 bis 12 und
- n: eine gerade Zahl m
bedeuten.

Ist R³ einer dieser Reste, so ist R⁴ bevorzugt Wasserstoff. Die Variable m kann dabei 1 bis 100 bedeuten. Bevorzugt ist m 1,2,3,4 oder eine Zahl von 10 bis 50, wobei besonders bei den oligomeren oder polymeren Resten R³ in der Regel Gemische eingesetzt werden.

Als R⁷ kommen die gleichen Reste in Betracht, wie sie für R⁵ genannt sind. Bevorzugt steht R⁷ für C₁-C₄-Alkyl.

Als R⁸ kommen neben Wasserstoff die gleichen Reste in Betracht, wie sie für R⁶ genannt worden sind. Bevorzugt steht R⁸ für Wasserstoff.

Als R⁹ kommen besonders Vinyl, Isopropenyl oder C₁₅-C₁₇-Alkylreste in Betracht.

Als R¹⁰ kommen beispielsweise die oben genannten C₈-C₁₈-Alkylreste sowie Nonadecyl, Eicosyl, Uneicosyl und Doeicosyl in Betracht. Dabei sind Mischungen verschiedener Reste R¹⁰, die sich in der Länge der Kohlenstoffkette unterscheiden, bevorzugt.

Die Reste R¹¹ sind Wasserstoff oder organische Reste, wie sie bei der radikalischen Polymerisation der Ausgangsmonomeren, in diesem Fall von einem Ethylenderivat und einem Maleinsäureimidderivat, entstehen, also z.B. ein Rest, der aus dem Polymerisationsinitiator oder aus einem intermediär aufgetretenen Radikal entsteht oder ein anderer derartiger Rest, wie er dem Fachmann geläufig ist.

Bevorzugte Nitroxylverbindungen sind auch die folgenden:
1-Oxyl-2,2,6,6-tetramethylpiperidin,
1-Oxyl-2,2,6,6-tetramethylpiperidin-4-ol,
1-Oxyl-2,2,6,6-tetramethylpiperidin-4-on,
1-Oxyl-2,2,6,6-tetramethylpiperidin-4-yl-acetat,
1-Oxyl-2,2,6,6-tetramethylpiperidin-4-yl-2-ethylhexanoat,
1-Oxyl-2,2,6,6-tetramethylpiperidin-4-yl-stearat,
1-Oxyl-2,2,6,6-tetramethylpiperidin-4-yl-benzoat,
1-Oxyl-2,2,6,6-tetramethylpiperidin-4-yl-(4-tert-butyl)benzoat,
Bis(1-oxyl-2,2,6,6-tetramethylpiperidin-4-yl)-succinat,
Bis(1-oxyl-2,2,6,6-tetramethylpiperidin-4-yl)-adipat,
Bis(1-oxyl-2,2,6,6-tetramethylpiperidin-4-yl)-sebacat,
Bis(1-oxyl-2,2,6,6-tetramethylpiperidin-4-yl)-n-butylmalonat,
Bis(1-oxyl-2,2,6,6-tetramethylpiperidin-4-yl)-phthalat,
Bis(1-oxyl-2,2,6,6-tetramethylpiperidin-4-yl)-isophthalat,
Bis(1-oxyl-2,2,6,6-tetramethylpiperidin-4-yl)-terephthalat,
Bis(1-oxyl-2,2,6,6-tetramethylpiperidin-4-yl)-hexyhydroterephthalat,
N,N'-Bis(1-oxyl-2,2,6,6-tetramethylpiperidin-4-yl)-adipinamid,
N-(1-Oxyl-2,2,6,6-tetramethylpiperidin-4-yl)-caprolactam,
N-(1-Oxyl-2,2,6,6-tetramethylpiperidin-4-yl)-dodecylsuccinimid,
2,4,6-Tris-[N-butyl-N-(1-oxyl-2,2,6,6,-tetramethylpiperidin-4-yl]-s-triazin,
4,4'-Ethylenbis(1-oxyl-2,2,6,6-tetramethylpiperazin-3-on) und
Tris-(2,2,6,6-tetramethyl-1-oxyl-piperidin-4-yl)phosphit.

Besonders bevorzugte N-Oxylderivate sind solche der allgemeinen Formel I 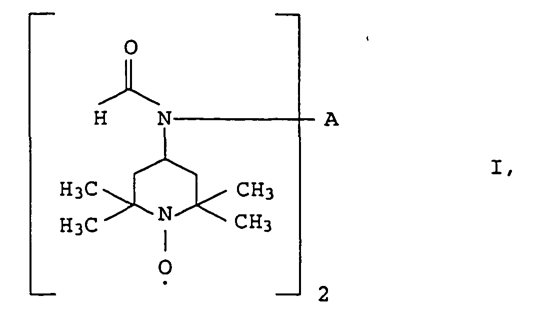 in der A einen zweiwertigen organischen Rest mit 2 bis 20 C-Atomen bedeutet.

Geeignete Reste A sind z.B. α,ω-Alkylengruppen, deren Kohlenstoffkette gerade oder verzweigt sein kann und durch Sauerstoffatome in Etherfunktion, Imino- oder C₁-C₄-Alkyliminogruppen unterbrochen sein kann. Solche Alkylengruppen werden im einzelnen z.B. in der älteren deutschen Patentanmeldung 19510184.7 genannt. Bevorzugte Reste A sind geradkettige α,ω-Alkylengruppen mit 2 bis 10 C-Atomen, besonders bevorzugt ist die Hexamethylengruppe.

Als geeignete Hydroxylaminderivate sterisch gehinderter Amine kommen alle diejenigen Strukturen in Betracht, wie sie als N-Oxyle genannt wurden, wobei die N-Oxyl-Gruppe jeweils durch eine hydroxylamino-Gruppe ersetzt ist.

In dem erfindungsgemäßen Verfahren können neben den Derivaten sterisch gehinderter Amine auch Costabilisatoren eingesetzt werden. Als Costabilisatoren kommen beispielsweise aromatische Nitro- oder Nitrosoverbindungen sowie Hydroxylamine, auch sterisch nicht gehinderte, in Betracht.

Als aromatische Nitroverbindungen können beispielsweise 1,3-Dinitrobenzol, 1,4-Dinitrobenzol, 2,6-Dinitro-4-methylphenol, 2-Nitro-4-methylphenol, 2,4,6-Trinitrophenol, 2,4-Dinitro-1-naphthol, 2,4-Dinitro-6-methylphenol, 2,4-Dinitrochlorbenzol, 2,4-Dinitrophenol, 2,4-Dinitro-6-sec-butylphenol, 4-Cyano-2-nitrophenol, 3-Iodo-4-cyano-5-nitrophenol, besonders bevorzugt 2,6-Dinitro-4-methylphenol, 2-Nitro-4-methylphenol, 2,4-Dinitro-6-sec-butylphenol bzw. 2,4-Dinitro-6-methylphenol verwendet werden.

Als aromatische Nitrosoverbindungen kommen z.B. p-Nitrosophenol, p-Nitroso-o-kresol und p-Nitroso-N,N'-diethylanilin in Betracht.

Weiterhin können als Costabilisatoren Verbindungen aus der Gruppe der Chinone, der Phenothiazine und der Phenole eingesetzt werden.

Geeignete substituierte Phenole sind beispielsweise die folgenden:
4-tert-Butylbrenzcatechin, Methoxyhydrochinon, 2,6-Di-tert-butyl-4-methylphenol, n-Octadecyl-β-(3,5-di-tert-butyl-4-hydroxyphenyl)-propionat, 1,1,3-Tris-(2-methyl-4-hydroxy-5-tert-butylphenyl)-butan, 1,3,5-Trimethyl-2,4,6-tris-(3,5-di-tert-butyl-4-hydroxybenzyl)-benzol, 1,3,5-Tris-(3,5-di-tert-butyl-4-hydroxybenzyl)-isocyanurat, 1,3,5-Tris-[β-(3,5-di-tert-butyl-4-hydroxyphenyl)-propionyloxyethyl-isocyanurat, 1,3,5-Tris-(2,6-dimethyl-3-hydroxy-4-tert-butylbenzyl)-isocyanurat und Pentaerythrittetrakis-[β-(3,5-di-tert-butyl-4-hydroxy-phenyl)-propionat].

Die sterisch gehinderten Aminderivate werden im erfindungsgemäßen Verdichtungsverfahren in einer Konzentration zwischen 0,00001 und 1 Gew.-%, bezogen auf die Menge der zu verdichtenden Monomeren, bevorzugt zwischen 0,0001 und 0,1 Gew.-% eingesetzt. Dieser Konzentrationsbereich gilt auch für die genannten Costabilisatoren.

Nach dem erfindungsgemäßen Verfahren werden die Monomeren auf einen Druck von 200 bis 5000 bar verdichtet. Die Verdichtung erfolgt vorzugsweise stufenweise, der Enddruck liegt vorzugsweise zwischen 1000 und 4000, besonders bevorzugt zwischen 1500 und 3000 bar.

Die Temperaturen bei der Verdichtung liegen vorzugsweise zwischen 20 und 140°C, besonders bevorzugt zwischen 30 und 100°C.

Während des Verdichtungsprozesses ist erfindungsgemäß kein Polymerisationsinitiator zugegen. Unter Polymerisationsinitiatoren sind dabei alle Verbindungen zu verstehen, die zur Initiierung der radikalischen Polymerisation den Monomeren zugesetzt werden, wie Azoverbindungen, organische Peroxide und Hydroperoxide. Eventuell im Verdichtungsgemisch anwesender Sauerstoff soll hier nicht als Polymerisationsinitiator verstanden werden.

Das erfindungsgemäße Verdichtungsverfahren ist vorzugsweise Teil eines Hochdruckcopolymerisationsverfahrens, welches dadurch gekennzeichnet ist, daß man die Comonomeren einzeln oder gemischt nach den erfindungsgemäßen Verfahren verdichtet und anschließend bei einer Temperatur zwischen 50 und 350°C durch Zugabe eines Polymerisationsinitiators polymerisiert.

Die bevorzugte Polymerisationstemperatur liegt zwischen 150 und 300°C, der bevorzugte Druck zwischen 1000 und 4000 bar, besonders bevorzugt zwischen 2000 und 3000 bar.

Die Polymerisation kann nach üblichen Methoden z.B. in Rohrreaktoren oder in Autoklavenreaktoren erfolgen. Es können alle üblichen Zusätze, beispielsweise Molekulargewichtsregler, Lösungsmittel usw., im Polymerisationsgemisch vorhanden sein.

Der Vorteil dieses erfindungsgemäßen Polymerisationsverfahrens liegt nicht nur in den längeren Laufzeiten der Verdichtungsapparaturen, sondern auch in einer Verbesserung der Polymerisationsprodukte. So neigt beispielsweise bei der Copolymerisation von Ethylen und Acrylsäure besonders die Acrylsäure zu vorzeitiger Polymerisation bei der Verdichtung. Dies führt zu Anteilen an Acrylsäurehomopolymeren oder zumindest von Homopolymerbereichen in den Copolymeren und damit zu beeinträchtigter Homogenität des Produktes und schlechterer Reproduzierbarkeit des Herstellverfahrens. Die nach dem erfindungsgemäßen Verfahren erhältlichen Copolymere weisen dagegen eine größere Homogenität auf.

### Beispiele

In einem Vorverdichter wurde Ethylen auf einen Druck von 220 bar komprimiert. In den komprimierten Gasstrom (1400 kg/Stunde) wurden in 60 Minuten 85 l eines Gemisches aus Acrylsäure und Isododecan (1:1, Vol:Vol), in welchem die in der Tabelle angegebenen Mengen des Stabilisators N,N'-Bis-(2,2,6,6-tetramethylpiperidin-1-oxyl-4-yl)-N,N'-bisformyl-hexamethylendiamin gelöst worden waren, gepumpt. Die Mischung wurde im Nachverdichter auf 2300 bar verdichtet und kontinuierlich in einen 35 l Stahldurchflußautoklaven überführt. Die Polymerisation wurde durch Zugabe von 0,0025 mol-% (bezogen auf die Gesamtmolmenge der Monomeren) tert-Butylperpivalat gestartet. Die Reaktionstemperatur betrug 220°C. Die Reaktion wurde beendet, wenn die auf Ablagerungen zurückzuführende Leckgasmenge am Nachverdichter 50 kg/Stunde überschritt.

Die Ergebnisse der Beispielversuche zeigt die folgende Tabelle:

| Beispiel | Inhibitorkonz. [Gew.-%] | Laufzeit [h] |
|---|---|---|
| 1 | 0,020 | > 168 |
| 2 | 0,010 | > 168 |
| 3 | 0,005 | 124 |
| Vergleich | 0 | 27 |

## Patentansprüche

1. Verfahren zum Verdichten von ethylenisch ungesättigten Monomeren auf einem Druck zwischen 200 und 5000 bar in Abwesenheit eines Polymerisationsinitiators, **dadurch gekennzeichnet, daß** die Verdichtung in Gegenwart eines sekundären Amins, das an den zum Aminstickstoff benachbarten C-Atomen so substituiert ist, daß es dort keine Wasserstoffatome trägt, vorgenommen wird.

2. Verfahren nach Anspruch 1, **dadurch gekennzeichnet, daß** als ethylenisch ungesättigte Monomere Gemische enthaltend Ethylen, Propylen oder Butadien eingesetzt werden.

3. Verfahren nach Anspruch 1 und 2, **dadurch gekennzeichnet, daß** als ethylenisch ungesättigte Monomere Gemische, welche Derivate der Acrylsäure oder Methacrylsäure oder diese Säuren selbst enthalten, eingesetzt werden.

4. Verfahren nach den Ansprüchen 1 bis 3, **dadurch gekennzeichnet, daß** als ethylenisch ungesättigte Monomere ein Gemisch enthaltend Ethylen und Acrylsäure eingesetzt wird.

5. Verfahren nach den Ansprüchen 1 bis 4, **dadurch gekennzeichnet, daß** die Verdichtung in Gegenwart eines N-Oxylderivats eines sekundären Amins, das an den zum Aminstickstoff benachbarten C-Atomen so substituiert ist, daß es dort keine Wasserstoffatome trägt, vorgenommen wird.

6. Verfahren nach den Ansprüchen 1 bis 5, **dadurch gekennzeichnet, daß** die Verdichtung in Gegenwart einer Verbindung der allgemeinen Formel I 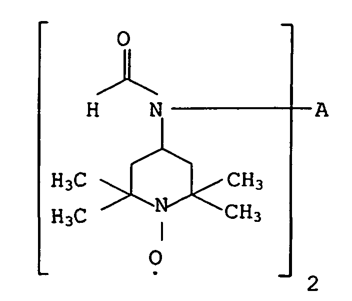 vorgenommen wird, in welcher A einen zweiwertigen organischen Rest mit 2 bis 20 C-Atomen bedeutet.

7. Verfahren nach den Ansprüchen 1 bis 6, **dadurch gekennzeichnet, daß** die Verdichtung in Anwesenheit von Hydroxylaminen oder von aromatischen Nitro- oder Nitrosoverbindungen vorgenommen wird.

8. Verfahren nach den Ansprüchen 1 bis 6, **dadurch gekennzeichnet, daß** die Verdichtung in Anwesenheit einer Verbindung aus der Gruppe der Chinone, der Phenothiazine und der Phenole vorgenommen wird.

9. Verfahren zur Herstellung von Copolymeren, **dadurch gekennzeichnet, daß** man die Comonomeren einzeln oder gemischt nach einem Verfahren gemäß den Ansprüchen 1 bis 8 verdichtet und anschließend bei einer Temperatur zwischen 50 und 350°C durch Zugabe eines Polymerisationsinitiators polymerisiert.

10. Verwendung von sekundären Aminen, die an den zum Aminstickstoff benachbarten C-Atomen so substituiert sind, daß sie dort keine Wasserstoffatome tragen, zur Verhinderung der vorzeitigen Polymerisation bei der Verdichtung ethylenisch ungesättigter Monomerer auf einen Druck zwischen 200 und 5000 bar.

## Claims

1. A process for compressing ethylenically unsaturated monomers at a pressure of 200 - 5000 bar in the absence of a polymerization initiator, which comprises carrying out compression in the presence of a secondary amine whose substituents on the carbons adjacent to the amine nitrogen ensure that no hydrogen is present thereon.

2. A process as claimed in claim 1, wherein mixtures comprising ethylene, propylene or butadiene are employed as ethylenically unsaturated monomers.

3. A process as claimed in claim 1 or 2, wherein mixtures comprising derivatives of acrylic or methacrylic acid or these acids themselves are employed as ethylenically unsaturated monomers.

4. A process as claimed in any of claims 1 to 3, wherein a mixture comprising ethylene and acrylic acid is employed as ethylenically unsaturated monomers.

5. A process as claimed in any of claims 1 to 4, wherein compression is carried out in the presence of an N-oxyl derivative of a secondary amine whose substituents on the carbons adjacent to the amine nitrogen ensure that no hydrogen is present thereon.

6. A process as claimed in any of claims 1 to 5, wherein compression is carried out in the presence of a compound of the formula I 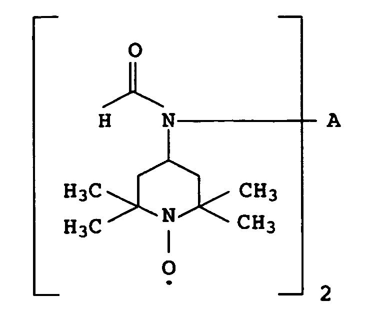 in which A is a divalent organic radical of 2 to 20 carbons.

7. A process as claimed in any of claims 1 to 6, wherein compression is carried out in the presence of hydroxylamines or of aromatic nitro or nitroso compounds.

8. A process as claimed in any of claims 1 to 6, wherein compression is carried out in the presence of a compound from the group consisting of the quinones, the phenothiazines and the phenols.

9. A process for preparing copolymers, which comprises compressing the comonomers, individually or as a mixture, in accordance with a process as claimed in any of claims 1 to 8, and then carrying out polymerization at from 50 to 350°C by adding a polymerization initiator.

10. The use of secondary amines whose substituents on the carbons adjacent to the amine nitrogen ensure that no hydrogen is present thereon, for preventing premature polymerization in the compression of ethylenically unsaturated monomers to a pressure of 200 - 5000 bar.

## Revendications

1. Procédé pour comprimer des monomères à insaturation éthyléniques à une pression comprise entre 200 et 5000 bar en l'absence d'un amorceur de polymérisation, **caractérisé en ce que** la compression est mise en oeuvre en présence d'une amine secondaire qui est substituée sur les atomes de carbone voisins de l'azote d'amine de façon qu'elle ne porte aucun atome d'hydrogène.

2. Procédé selon la revendication 1, **caractérisé en ce qu'**il utilise en tant que monomères à insaturation éthylénique des mélanges contenant de l'éthylène, du propylène ou du butadiène.

3. Procédé selon les revendications 1 et 2, **caractérisé en ce qu'**il utilise en tant que monomères à insaturation éthylénique des mélanges qui contiennent des dérivés de l'acide acrylique ou de l'acide méthacrylique, ou ses acides proprement dits.

4. Procédé selon les revendications 1 à 3, **caractérisé en ce qu'**il utilise en tant que monomères à insaturation éthylénique un mélange contenant de l'éthylène et de l'acide acrylique.

5. Procédé selon les revendications 1 à 4, **caractérisé en ce que** la compression est mise en oeuvre en présence d'un dérivé N-oxyle d'une amine secondaire qui est substituée sur les atomes de carbone voisins de l'azote d'amine de façon qu'elle ne porte aucun atome d'hydrogène.

6. Procédé selon les revendications 1 à 5, **caractérisé en ce que** la compression est mise en oeuvre en présence d'un composé de formule générale I 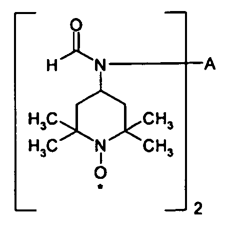 dans laquelle A est un résidu organique divalent ayant de 2 à 20 atomes de carbone.

7. Procédé selon les revendications 1 à 6, **caractérisé en ce que** la compression est mise en oeuvre en présence d'hydroxylamines ou de composés nitro ou nitroso aromatiques.

8. Procédé selon les revendications 1 à 6, **caractérisé en ce que** la compression est mise en oeuvre en présence d'un composé du groupe des quinones, des phénothiazines et des phénols.

9. Procédé de préparation de copolymères, **caractérisé en ce qu'**on comprime les comonomères, à titre individuel ou en mélange, par un procédé selon les revendications 1 à 8, puis on les polymérise à une température comprise entre 50 et 350°C par addition d'un amorceur de polymérisation.

10. Utilisation d'amines secondaires qui sont substituées sur les atomes de carbone voisins de l'azote d'amine de façon qu'elles ne portent aucun atome d'hydrogène, pour empêcher une polymérisation prématurée lors de la compression de monomères à insaturation éthylénique à une pression comprise entre 200 et 5000 bar.
